# EUROPÄISCHE PATENTSCHRIFT

(11) **EP 2 007 349 B1**
(45) Veröffentlichungstag und Bekanntmachung des Hinweises auf die Patenterteilung: **02.08.2017**
(21) Anmeldenummer: 07727802.6
(22) Anmeldetag: 04.04.2007
(51) Int. Cl.: A61K 9/00, A61K 9/12, A61K 9/72, A61K 31/00, A61K 47/06

(54) **AEROSOLSUSPENSIONSFORMULIERUNGEN MIT TG 227 EA ODER TG 134 A ALS TREIBMITTEL**
AEROSOL SUSPENSION FORMULATIONS COMPRISING TG 227 EA OR TG 134 A AS PROPELLANTS
FORMULATIONS DE SUSPENSIONS POUR AÉROSOLS UTILISANT DU TG 227 EA OU DU TG 134 A COMME PROPULSEUR

(30) Priorität: 11.04.2006 DE 102006017320
(43) Veröffentlichungstag der Anmeldung: 31.12.2008
(73) Patentinhaber: Boehringer Ingelheim Pharma GmbH & Co. KG, 55216 Ingelheim am Rhein (DE)
(72) Erfinder: HOELZ, Hubert, 55413 Oberheimbach (DE); MANN, Mariola, 55411 Bingen (DE); SCHMELZER, Christel, 55218 Ingelheim Am Rhein (DE); SCHMIDT, Friedrich, 55218 Ingelheim Am Rhein (DE); WEIL, Hans-Hermann, 55599 Gau-bickelheim (DE)
(74) Vertreter: Simon, Elke Anna Maria
(86) Internationale Anmeldenummer: PCT/EP2007/053333
(87) Internationale Veröffentlichungsnummer: WO 2007/118802

(56) Entgegenhaltungen:
- WO-A-02/17882
- WO-A-2006/002840
- WO-A-2006/064283
- DE-A1-102004 032 322
- US-A- 6 123 924

## Beschreibung

Die Erfindung betrifft Druckgaszubereitungen für Dosieraerosole, bei denen ein Arzneimittel in TG 227 ea (1,1,1,2,3,3,3-Heptafluorpropan) und/oder TG 134 a (1,1,1,2-Tetrafluorethan) als Treibmittel suspendiert formuliert ist, sowie deren Verwendung zur Herstellung eines Arzneimittels. Bevorzugt handelt es sich um ein Inhalationsaerosol.

### Stand der Technik

Seit Anfang der 1990er Jahre ist bekannt, dass die Treibgase TG 227 ea oder TG 134 a als alternative Treibgase für Fluorchlorkohlenwasserstoffe in Inhalationsaerosolen eingesetzt werden können.

Überraschend hat sich nun herausgestellt, dass Treibgasformulierungen mit suspendierten Wirkstoffpartikeln und TG 227 ea und/oder TG 134 a als Treibmittel eine verminderte Entmischung der Wirkstoffe in der Suspension zeigen, wenn spezielle oberflächenaktive Stoffe (Surfactants) zum Einsatz kommen.

### Detaillierte Beschreibung der Erfindung

Für die erfindungsgemäßen Treibmittelformulierungen werden als Treibgase TG 227 ea und/oder TG 134 a eingesetzt, gegebenenfalls in Mischung mit einem oder mehreren weiteren Treibgasen, vorzugsweise ausgewählt aus der Gruppe bestehend aus Propan, Butan, Pentan, Dimethylether, CHClF₂, CH₂F₂, CF₃CH₃, Isobutan, Isopentan und Neopentan.

Erfindungsgemäß bevorzugt sind solche Suspensionen, die als Treibgas nur TG 227 ea oder nur TG 134 a enthalten.
Wird in den erfindungsgemäßen Suspensionsformulierungen ein Gemisch der Treibgase TG 227 ea und TG 134a eingesetzt, so sind die Gewichtsverhältnisse, in denen diese beiden Treibgaskomponenten zum Einsatz gelangen können, frei variabel, wobei TG 227 ea vorhanden sein muss.
In Gemischen mit einem oder mehreren weiteren Treibgasen, ausgewählt aus der Gruppe bestehend aus Propan, Butan, Pentan, Dimethylether, CHClF₂, CH₂F₂, CF₃CH₃, Isobutan, Isopentan und Neopentan eingesetzt, liegt der Anteil dieser weiteren Treibgaskomponente vorzugsweise unter 60 %, bevorzugt unter 40% besonders bevorzugt unter 30%.

Als Wirkstoffe werden bevorzugt Wirkstoffe eingesetzt, die ein oder mehrere Wassermoleküle in ihrer Partikelstruktur einlagern oder binden. Dabei ist das Wasser nicht lediglich physikalisch mit den Wirkstoffpartikeln vermengt. Bevorzugt handelt es sich bei den Wirkstoffpartikeln um Kristalle und bei dem Wasser um Kristallwasser oder komplexgebundenes Wasser oder anderweitig chemisch gebundenes Wasser, z.B. Hydrate. Diese Form der Wassereinlagerung wird im Folgenden auch chemisch gebundenes Wasser genannt. In diesen Fällen hat das Wasser in der Regel auch Einfluss auf die Kristallstruktur des Wirkstoffmoleküls.

Bevorzugt werden inhalativ wirksame Verbindungen eingesetzt, mit der Folge, dass die erfindungsgemäßen Suspensionsformulierungen bevorzugt zur Inhalation bestimmt sind.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 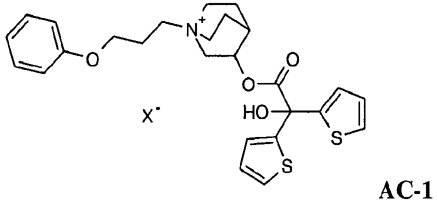 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 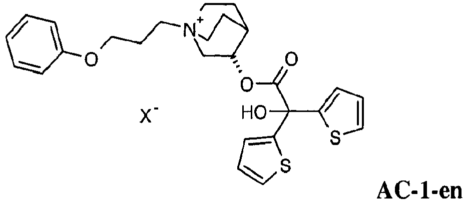 enthalten, worin X⁻ die vorstehend genannten Bedeutungen aufweisen kann. Weiterhin bevorzugte Anticholinergika sind ausgewählt aus den Salzen der Formel **AC-2** 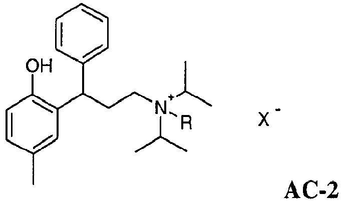 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC**-**2**-**base** vorliegen.

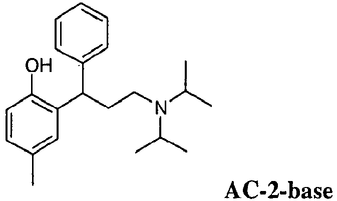

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino }-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy }-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Der Anteil des suspendierten Arzneistoffs an der fertigen Zubereitung beträgt zwischen 0,001 und 5 %, vorzugsweise 0,005 bis 3 %, insbesondere 0,01 bis 2 % (% = Gewichtsprozent).

Im Fall von Ipratropiumbromid-Monohydrat enthalten die erfindungsgemäßen Suspensionen vorzugsweise zwischen 0,001 bis 1%, besonders 0,005 bis 0,5% Ipratropium. Erfindungsgemäß besonders bevorzugt sind Suspensionen, die 0,01 bis 0,1% Ipratropium enthalten.

Im Fall von Salbutamol und seinen Salzen enthalten die erfindungsgemäßen Suspensionen vorzugsweise zwischen 0,005 bis 5%, besonders 0,025 bis 2,5% Salbutamol. Erfindungsgemäß besonders bevorzugt sind Suspensionen, die 0,05 bis 1% Salbutamol enthalten.

Im Fall von Tiotropiumbromid-Monohydrat enthalten die erfindungsgemäßen Suspensionen vorzugsweise zwischen 0,001 bis 1%, besonders 0,0012 bis 0,8% Tiotropium. Erfindungsgemäß bevorzugt sind Suspensionen, die 0,002 bis 0,5%, besonders bevorzugt 0,008 bis 0,4% Tiotropium enthalten.

Für die o.g. Konzentrationsangaben der Wirkstoffe ist unter Tiotropium und Ipratropium jeweils das freie Ammoniumkation zu verstehen; Unter Salbutamol ist die Salbutamol-Base zu verstehen.

Die erfindungsgemäßen Treibgas-Suspensionen sind dadurch gekennzeichnet, dass sie Tiotropium oder Ipratropium in Form der kristallinen Monohydrate enthalten. Dementsprechend betrifft die vorliegende Erfindung vorzugsweise Suspensionen, die kristallines Tiotropiumbromid-Monohydrat oder Ipratropiumbromid-Monohydrat enthalten.

Bei den im Rahmen der vorliegenden Erfindung genannten prozentualen Angaben, handelt es sich stets um Massenprozente. Werden Massenanteile für Tiotropium in Massenprozenten zum Ausdruck gebracht, sind die entsprechenden Werte für das im Rahmen der vorliegenden Erfindung bevorzugt zum Einsatz gelangende kristalline Tiotropiumbromid-Monohydrat durch Multiplikation mit dem Umrechnungsfaktor 1,2495 erhältlich. Analoges gilt für Ipratopium.

Die erfindungsgemäßem treibgashaltigen Inhalationsaerosole bzw. Suspensionsformulierungen können weitere Bestandteile wie oberflächenaktive Mittel (Tenside, Surfactants), Adjuvantien, Antioxidantien oder Geschmacksmittel enthalten.

Die in den erfindungsgemäßen Suspensionen enthaltenen oberflächenaktiven Mittel (Tenside, Surfactants) sind vorzugsweise ausgewählt aus der Gruppe bestehend aus Polyethylenglycolen (PEG) und/oder Polyvinylpyrrolidonen (PVP, Povidon) und/oder Isopropylmyristat. Von den vorstehend genannten Suspensionshilfsstoffen gelangen bevorzugt zur Anwendung PEG 200, PEG 400 und/oder das Polyvinylpyrrolidon K25 und/oder Isopropylmyristat.
Sofern in den erfindungsgemäßen Suspensionen oberflächenaktive Mittel enthalten sind, werden diese vorzugsweise in einem Anteil von 0,005 - 5 %, besonders bevorzugt 0,01 - 1 % eingesetzt.

Werden wasserfreie Treibgase eingesetzt, wird diesen eine geringe Menge an Wasser beigemischt. Es können aber auch wasserhaltige Treibgase eingesetzt werden, wobei diese bei ihrer Verwendung einen bestimmten Wassergehalt aufweisen sollten. Dieses zugesetzte bzw. vorhandene Wasser ist in der fertigen Suspensionsformulierung verschieden von Wasser, welches in einem der Wirkstoffe oder den Hilfsstoffen chemisch gebunden vorliegt. Dieses nicht chemisch gebundene Wasser wird auch als freies Wasser bezeichnet, um es von dem mit dem Wirkstoff molekular bzw. chemisch verbundenem Wasser abzugrenzen.

Es hat sich gezeigt, dass sich die suspendierten Wirkstoffpartikel bei einem zu geringen Wasseranteil verändern. Andererseits hat sich gezeigt, dass sich die Partikelgrößen bei einem zu hohen Wassergehalt ebenfalls verändern. Der optimale Wassergehalt kann für jede Substanz individuell bestimmt werden. Dabei hat sich gezeigt, dass die bevorzugte Wassermenge im Allgemeinen in dem Treibgas TG 227 ea oder in Mischungen aus TG 227 ea mit Treibgasen aus der Gruppe Propan, Butan, Pentan, Dimethylether, CHClF₂, CH₂F₂, CF₃CH₃, Isobutan, Isopentan und Neopentan, 10 bis 1000 ppm beträgt, besonders bevorzugt 50 bis 500 ppm und ganz besonders bevorzugt liegt die Wassermenge bei 100 bis 450 ppm.

Im Fall von Ipratropiumbromid-Monohydrat haltigen Formulierungen mit Treibgas TG 227 ea beträgt der am stärksten bevorzugte Wassergehalt der Formulierung zwischen 20 und 500 ppm, insbesondere liegt der Wassergehalt zwischen 50 und 350 ppm.

Im Fall von Tiotropiumbromid-Monohydrat liegt der bevorzugte Wassergehalt bei vergleichbaren Größen wie für Ipratropiumbromid-Monohydrat. Der am stärksten bevorzugte Bereich liegt zwischen 50 und 230 ppm.

Es hat sich außerdem gezeigt, dass die bevorzugte Wassermenge in dem Treibgas TG 134 a oder in Mischungen aus TG 134 a mit Treibgasen aus der Gruppe Propan, Butan, Pentan, Dimethylether, CHClF₂, CH₂F₂, CF₃CH₃, Isobutan, Isopentan und Neopentan zwischen 30 und 4000 ppm, besonderns bevorzugt zwischen 150 und 2000 ppm und ganz besonders bevorzugt zwischen 350 und 1700 ppm liegt.

Im Fall von Ipratropium-Monohydrat haltigen Formulierungen mit Treibgas TG 134 a beträgt der am stärksten bevorzugte Wassergehalt der Formulierung zwischen 70 und 1800 ppm, insbesondere liegt der Wassergehalt zwischen 180 und 1300 ppm.

Im Fall von Tiotropium-Monohydrat liegt der bevorzugte Wassergehalt bei vergleichbaren Größen wie für Ipratropiumbromid. Der am stärksten bevorzugte Bereich liegt zwischen 180 und 900 ppm.

Werden Mischungen aus den Treibgasen TG 134 a und TG 227 ea verwendet, so ergeben sich die bevorzugten Wassergehälter aus dem Mischungsverhältnis der beiden Treibgase.

Erfindungsgemäß werden den Treibgasen oder den fertigen Aerosolsuspensionen bevorzugt diese Mengen Wasser zugesetzt, wenn das Treibgas, Treibgasgemisch oder die Formulierung bis auf das chemisch an den Wirkstoff gebundene Wasser kein sonstiges Wasser enthält (freies Wasser). Verfahrenstechnisch kann dabei das Wasser bereits dem Treibgas zugemischt werden, bevor die Arzneimittelsuspension hergestellt wird oder zunächst wird die Arzneimittelsuspension mit wasserfreiem Treibgas oder Treibgasgemisch hergestellt und anschließend wird die entsprechende Menge Wasser beigemischt.
Die ppm-Angaben beziehen sich als Bezugsgröße auf das verflüssigte Treibmittel.

Gegebenenfalls wird im Rahmen der vorliegenden Erfindung statt des Begriffs Suspension auch der Begriff Suspensionsformulierung verwendet. Beide Begriffe sind im Rahmen der vorliegenden Erfindung als gleichbedeutend anzusehen.

Im Hinblick auf die inhalative Applikation ist es erforderlich, den Wirkstoff in feinteiliger Form bereitzustellen. Der Wirkstoff wird dazu entweder gemahlen (mikronisiert) oder über andere technische, im Stand der Technik prinzipiell bekannte Verfahren (beispielsweise Präzipitation, Sprühtrocknung) in feinteiliger Form gewonnen.
Verfahren zur Mikronisierung von Wirkstoffen sind im Stand der Technik bekannt. Vorzugsweise weist der Wirkstoff nach Mikronisierung eine mittlere Teilchengröße von 0,1 bis 10*µ*m, bevorzugt von 0,5 bis 6*µ*m, besonders bevorzugt von 1 bis 5*µ*m auf.

Zur Herstellung der erfindungsgemäßen Suspensionen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Die vorstehend genannten erfindungsgemäßen treibgashaltigen Suspensionen können mittels im Stand der Technik bekannten Inhalatoren (pMDIs = pressurized metered dose inhalers) appliziert werden. Dementsprechend betrifft ein weiterer Aspekt der vorliegenden Erfindung Arzneimittel in Form von wie vorstehend beschriebenen Suspensionen in Verbindung mit einem oder mehreren zur Verabreichung dieser Suspensionen geeigneten Inhalatoren. Ferner betrifft die vorliegende Erfindung Inhalatoren, dadurch gekennzeichnet, dass sie vorstehend beschriebene erfindungsgemäße treibgashaltige Suspensionen enthalten.
Die vorliegende Erfindung betrifft ferner Behälter (z.B. Kartuschen), die mit einem geeigneten und vor der Verwendung bzgl. des Wassergehaltes konditionierten Ventil ausgestattet sein können.
Die Behälter können in einem geeigneten Inhalator zur Anwendung gelangen und eine der vorstehend genannten erfindungsgemäßen treibgashaltigen Suspensionen enthalten. Geeignete Behälter (z.B. Kartuschen) und Verfahren zur Abfüllung dieser Kartuschen mit den erfindungsgemäßen treibgashaltigen Suspensionen sind aus dem Stand der Technik bekannt.

Aufgrund der pharmazeutischen Wirksamkeit von Anticholinergika betrifft die vorliegende Erfindung ferner die Verwendung der erfindungsgemäßen Suspensionen zur Herstellung eines inhalativ oder nasal applizierbaren Arzneimittels, bevorzugt zur Herstellung eines Arzneimittels zur inhalativen oder nasalen Behandlung von Erkrankungen, in denen Anticholinergika einen therapeutischen Nutzen entfalten können.

Besonders bevorzugt betrifft die vorliegende Erfindung ferner die Verwendung der erfindungsgemäßen Suspensionen zur Herstellung eines Arzneimittels zur inhalativen Behandlung von Atemwegserkrankungen, bevorzugt von Asthma, COPD, Mucoviszidose, cystische Fibrose; weiterhin von systemischen Erkrankungen, wie Schmerz, Migräne Bluthochdruck, Erektionsstörungen.

Die nachfolgenden Beispiele dienen der exemplarischen, weitergehenden Illustration der vorliegenden Erfindung, ohne selbige auf deren Inhalt zu beschränken.

### Formulierungsbeispiele

Die Beispielformulierungen enthalten zusätzlich zu den im Einzelnen aufgeführten Inhaltsstoffen jeweils zwischen 100 und 350 ppm Wasser.

**Beispiel 1:**

| **Bestandteil** | **Gewichtsprozent** | **g/Behälter** |
|---|---|---|
| Salbutamolsulfat | 0,171 | 0,0312 |
| Ipratropiumbromid Monohydrat | 0,030 | 0,0055 |
| Polyvinylpyrrolidon K25 | 0,100 | 0,0183 |
| TG 227 ea | 99,700 | 18,244 |
| Summe | 100,000 | 18,2987 |

**Beispiel 2**

| **Bestandteil** | **Gewichtsprozent** | **g**/**Behälter** |
|---|---|---|
| Salbutamolsulfat | 0,171 | 0,0312 |
| Ipratropiumbromid Monohydrat | 0,030 | 0,0055 |
| Polyvinylpyrrolidon K25 | 0,100 | 0,0183 |
| Isopropylmyristat | 0,300 | 0,0548 |
| TG 227 ea | 99,399 | 18,1531 |
| Summe | 100,000 | 18,2628 |

**Beispiel 3:**

| **Bestandteil** | **Gewichtsprozent** | **g**/**Behälter** |
|---|---|---|
| Salbutamolsulfat | 0,171 | 0,0312 |
| Ipratropiumbromid Monohydrat | 0,030 | 0,0055 |
| Polyvinylpyrrolidon K25 | 0,100 | 0,0183 |
| Polyethylenglycol 200 | 0,300 | 0,0549 |
| TG 227 ea | 99,399 | 18,1751 |
| Summe | 100,000 | 18,2849 |

**Beispiel 4:**

| **Bestandteil** | **Gewichtsprozent** | **g**/**Behälter** |
|---|---|---|
| Salbutamolsulfat | 0,178 | 0,0312 |
| Ipratropiumbromid Monohydrat | 0,031 | 0,0055 |
| Polyvinylpyrrolidon K25 | 0,100 | 0,0176 |
| Polyethylenglycol 400 | 0,300 | 0,0527 |
| TG 134a | 28,000 | 4.9145 |
| TG 227 ea | 70,391 | 12,5304 |
| Summe | 100,000 | 17,5517 |

**Beispiel 5:**

| **Bestandteil** | **Gewichtsprozent** | **g**/**Behälter** |
|---|---|---|
| Salbutamolsulfat | 0,213 | 0,0312 |
| Ipratropiumbromid Monohydrat | 0,037 | 0,0055 |
| Polyvinylpyrrolidon K25 | 0,100 | 0,0146 |
| TG 227 ea | 99,650 | 14,5873 |
| Summe | 100,000 | 14,6386 |

**Beispiel 6**

| **Bestandteil** | **Gewichtsprozent** | **g/Behälter** |
|---|---|---|
| Salbutamolsulfat | 0,214 | 0,0312 |
| Ipratropiumbromid Monohydrat | 0,037 | 0,0055 |
| Polyvinylpyrrolidon K25 | 0,100 | 0,0146 |
| Isopropylmyristat | 0,300 | 0,0438 |
| TG 227 ea | 99,349 | 14,5147 |
| Summe | 100,000 | 14,6098 |

**Beispiel 7:**

| **Bestandteil** | **Gewichtsprozent** | **g**/**Behälter** |
|---|---|---|
| Salbutamolsulfat | 0,213 | 0,0312 |
| Ipratropiumbromid Monohydrat | 0,037 | 0,0055 |
| Polyvinylpyrrolidon K25 | 0,100 | 0,0146 |
| Polyethylenglycol 200 | 0,300 | 0,0439 |
| TG 227 ea | 99,350 | 14,5323 |
| Summe | 100,000 | 14,6275 |

**Beispiel 8:**

| **Bestandteil** | **Gewichtsprozent** | **g**/**Behälter** |
|---|---|---|
| Salbutamolsulfat | 0,222 | 0,0312 |
| Ipratropiumbromid Monohydrat | 0,039 | 0,0055 |
| Polyvinylpyrrolidon K25 | 0,100 | 0,0140 |
| Polyethylenglycol 400 | 0,300 | 0,0421 |
| TG 134a | 28,000 | 3,9315 |
| TG 227 ea | 71,339 | 10,0167 |
| Summe | 100,000 | 14,0410 |

## Patentansprüche

1. Treibmittelhaltige Aerosolsuspension enthaltend Wirkstoffpartikel mit chemisch gebundenem Wasser, wenigstens 85 Gew.% eines Treibgases oder eines Treibgasgemisches mit TG 227 ea gegebenenfalls im Gemisch mit wenigstens einem weiterem Treibgas ausgewählt aus der Gruppe bestehend aus Propan, Butan, Pentan, Dimethylether, CHClF₂, CH₂F₂, CF₃CH₃, Isobutan, Isopentan und Neopentan, **dadurch gekennzeichnet, dass** die Aerosolsuspension die oberflächenaktive Substanz Polyvinylpyrrolidon K25 in einem Anteil von 0,005 - 1 Gew.% enthalt, und dass die Suspension Tiotropiumbromid-Monohydrat als Wirkstoff enthält, wobei der Anteil des Tiotropiums zwischen 0,008 und 0,4 Gewichtsprozent beträgt und die Menge an Wasser in der Formulierung zwischen 50 und 230 ppm liegt.

2. Aerosolsuspension nach Anspruch 1, dadurch gekennzeichet, dass sie als weitere Bestandteile oberflächenaktive Mittel, Adjuvantien, Antioxidantien und/oder Geschmacksmittel enthalten.

3. Aerosolsuspension nach einem der vorangegangenen Ansprüche, **dadurch gekennzeichnet, dass** sie als Adjuvantien eine oder mehrere Verbindungen ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure enthalten.

4. Aerosolsuspension nach einem der vorangegangenen Ansprüche, dadurch gekennzeichet, dass sie als Antioxidantien eine oder mehrere Verbindungen ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat enthalten.

5. Verfahren zur Herstellung von Aerosolsuspensionen gemäß einem der Ansprüche 1 bis 4, **dadurch gekennzeichnet, dass**
(i) die Suspensionspartikel in feinteiliger Form aus Wirkstoff und Hilfsstoff durch Präzipitation oder Sprühtrocknung gewonnen werden,
(ii) danach mit dem oder den Treibgasen gemischt werden,
(iii) und die Suspension in geeignete Behälter abgefüllt wird.

6. Verfahren zur Herstellung von Aerosolsuspensionen gemäß einem der Ansprüche 1 bis 4, **dadurch gekennzeichnet, dass** wasserhaltiges Treibgas oder Treibgasgemisch eingesetzt wird, um die Aerosolsuspension herzustellen.

7. Verfahren zur Herstellung von Aerosolsuspensionen gemäß einem der Ansprüche 1 bis 4, **dadurch gekennzeichnet, dass** mit wasserfreiem Treibgas oder Treibgasgemisch eine Aerosolsuspension hergestellt wird und anschließend Wasser beigemischt wird.

8. Behälter enthaltend ein treibmittelhaltiges Aerosol nach einem oder mehreren der Ansprüche 1-4, **dadurch gekennzeichnet, dass** der Behälter ein geeignetes Ventil enthalt, welches vor der Verwendung bezüglich seines Wassergehaltes konditioniert wird.

## Claims

1. Propellant-containing aerosol suspension containing particles of active substance with chemically bound water, at least 85 wt.% of a propellant gas or a propellant gas mixture with TG 227 ea optionally in admixture with at least one other propellant gas selected from the group consisting of propane, butane, pentane, dimethyl ether, CHClF₂, CH₂F₂, CF₃CH₃, isobutane, isopentane and neopentane, **characterised in that** the aerosol suspension contains the surface-active substance polyvinylpyrrolidone K25 in a proportion of from 0.005 to 1 wt.%, and **in that** the suspension contains tiotropium bromide monohydrate as the active substance, the proportion of the tiotropium being between 0.008 and 0.4 wt.% and the amount of water in the formulation being between 50 and 230 ppm.

2. Aerosol suspension according to claim 1, **characterised in that** it contains as further ingredients surface-active substances, adjuvants, antioxidants and/or flavourings.

3. Aerosol suspension according to one of the preceding claims, **characterised in that** it contains as adjuvants one or more compounds selected from the group consisting of alanine, albumin, ascorbic acid, aspartame, betaine, cysteine, phosphoric acid, nitric acid, hydrochloric acid, sulphuric acid and citric acid.

4. Aerosol suspension according to one of the preceding claims, **characterised in that** it contains as antioxidants one or more compounds selected from the group consisting of ascorbic acid, citric acid, sodium edetate, editic acid, tocopherols, butylhydroxytoluene, butylhydroxyanisol and ascorbyl palmitate.

5. Process for preparing aerosol suspensions according to one of claims 1 to 4, **characterised in that**
(i) the suspension particles are obtained in particulate form from the active substance and auxiliary agent by precipitation or spray drying,
(ii) subsequently mixed with the propellant gas or gases,
(iii) and the suspension is filled into suitable containers.

6. Process for preparing aerosol suspensions according to one of claims 1 to 4, **characterised in that** water-containing propellant gas or propellant gas mixture is used to prepare the aerosol suspension.

7. Process for preparing aerosol suspensions according to one of claims 1 to 4, **characterised in that** an aerosol suspension is prepared with anhydrous propellant gas or propellant gas mixture and then water is added.

8. Container containing a propellant-containing aerosol according to one or more of claims 1 to 4, **characterised in that** the container contains a suitable valve which is adjusted before use in relation to its water content.

## Revendications

1. Suspension aérosol contenant un agent propulseur, comprenant des particules de principe actif comprenant de l'eau chimiquement liée, au moins 85 % en poids d'un gaz propulseur ou d'un mélange de gaz propulseurs comprenant du TG 227 ea éventuellement dans le mélange avec au moins un autre gaz propulseur choisi parmi le groupe constitué de propane, de butane, de pentane, de diméthyléther, de CHClF₂, de CH₂F₂, de CF₃CH₃, d'isobutane, d'isopentane et de néopentane, **caractérisée en ce que** la suspension aérosol comprend la substance tensioactive, la polyvinylpyrrolidone K25, en une proportion allant de 0,005 - 1 % en poids, et **en ce que** la suspension comprend en tant que principe actif du bromure de tiotropium monohydraté, la proportion de tiotropium étant comprise entre 0,008 et 0,4 pourcent en poids et la quantité en eau présente dans la formulation étant comprise entre 50 et 230 ppm.

2. Suspension aérosol selon la revendication 1, **caractérisée en ce qu'**elle comprend en tant qu'autres constituants des agents tensioactifs, des adjuvants, des antioxydants et/ou des agents aromatiques.

3. Suspension aérosol selon l'une quelconque des revendications précédentes, **caractérisée en ce qu'**elle comprend en tant qu'adjuvants un ou plusieurs composés choisis parmi le groupe constitué d'alanine, d'albumine, d'acide ascorbique, d'aspartame, de bétaïne, de cystéine, d'acide phosphorique, d'acide nitrique, d'acide chlorhydrique, d'acide sulfurique et d'acide citrique.

4. Suspension aérosol selon l'une quelconque des revendications précédentes, **caractérisée en ce qu'**elle comprend en tant qu'antioxydants un ou plusieurs composés choisis parmi le groupe constitué d'acide ascorbique, d'acide citrique, d'édétate de sodium, d'acide édétique, de tocophérols, d'hydroxytoluène butylé, d'hydroxyanisole butylé et de palmitate d'ascorbyle.

5. Procédé de préparation des suspensions aérosols selon l'une quelconque des revendications 1 à 4, **caractérisé en ce que**
(i) les particules de suspension présentes sous une forme fine sont obtenues à partir d'un principe actif et d'un excipient par précipitation ou par séchage par pulvérisation ;
(ii) elles sont mélangées par la suite au ou aux gaz propulseurs ;
(iii) et la suspension est transvasée dans des récipients adaptés.

6. Procédé de préparation des suspensions aérosols selon l'une quelconque des revendications 1 à 4, **caractérisé en ce qu'**est employé du gaz propulseur ou un mélange de gaz propulseurs contenant de l'eau afin de fabriquer la suspension aérosol.

7. Procédé de préparation des suspensions aérosols selon l'une quelconque des revendications 1 à 4, **caractérisé en ce qu'**une suspension aérosol est fabriquée avec du gaz propulseur ou un mélange de gaz propulseurs sans eau, et **en ce que** de l'eau est ajoutée immédiatement après.

8. Récipient contenant un aérosol contenant un agent propulseur selon l'une quelconque ou plusieurs des revendications précédentes, **caractérisé en ce que** le récipient contient une soupape adaptée, qui est conditionnée par rapport à sa teneur en eau avant l'utilisation.
